# EUROPEAN PATENT APPLICATION

(11) **EP 3 453 411 A1**
(43) Date of publication of application: **13.03.2019**
(21) Application number: 18275128.9
(22) Date of filing: 21.08.2018
(51) Int. Cl.: A61L 31/02, A61L 31/14

(54) **ENDOVASCULAR DEVICE CONFIGURED FOR CONTROLLED SHAPE MEMORY DEPLOYMENT IN A BODY VESSEL**

(30) Priority: 08.09.2017 US 201715699389
(71) Applicant: Cook Medical Technologies LLC, Bloomington, IN 47404 (US)
(72) Inventor: KIM, Woong, West Lafayette, IN 47906 (US)
(74) Representative: Williams Powell

(57) **Abstract**

A method of controllably deploying an endovascular device comprises delivering, into a body vessel, a Nitinol structural element (102) comprising a variable austenite finish temperature A_{f}(*x*) along a predetermined length (*L*) thereof, where 0 < x ≤ L. The variable austenite finish temperature A_{f}(*x*) increases or decreases monotonically as a function of *x* and lies above body temperature at any location along the predetermined length of the Nitinol structural element (102). During and/or after delivery into the body vessel, the Nitinol structural element (102) is heated above body temperature. As a temperature of the Nitinol structural element (102) reaches A_{f}(*x*) at each location along the predetermined length, the Nitinol structural element (102) recovers a pre-set shape at the respective location, and the endovascular device is controllably deployed.

## Description

### TECHNICAL FIELD

The present disclosure is related generally to endovascular devices and more specifically to an endovascular device comprising a nickel-titanium shape memory alloy ("Nitinol").

### BACKGROUND

Superelastic deployment of Nitinol-based endovascular devices is widely used to implant stents, filters and other devices into blood vessels. Such devices are typically heat set to a single static shape (e.g., a radially-expanded shape in the case of a stent) that can be recovered spontaneously upon removal of a constraining force, such as an overlying tubular sheath, after delivery of the device into a target vessel. Such nitinol-based devices may have austenite finish temperatures (A_{f}) below body temperature to ensure that removal of the constraining force, once the device is delivered into the vessel, is sufficient to induce the transformation from martensite to austenite that is needed for shape recovery. Shape memory deployment of endovascular devices, where austenite finish temperatures may be at or above body temperature and heating is employed to induce shape recovery, is not widely used for Nitinol-based endovascular devices due to a number of practical challenges, such as the difficulty of controlling temperature *in situ.* Furthermore, current Nitinol-based endovascular devices utilize a bimodal approach of deformation and recovery to a preset shape defined by a single A_{f} temperature.

### BRIEF SUMMARY

Aspects of the present invention seek to provide an improved device and/or method.

According to an aspect of the invention, there is provided an endovascular device as in claim 1.

According to an aspect of the invention, there is provided a method as in claim 7.

In an embodiment, an endovascular device configured for controlled deployment in a body vessel comprises a Nitinol structural element having a variable austenite finish temperature A_{f}(*x*) along a predetermined length (*L*) thereof, where 0 < x ≤ *L*. The variable austenite finish temperature A_{f}(*x*) monotonically increases or decreases as a function of x and lies above body temperature at any location along the predetermined length. Accordingly, the endovascular device is configured for controlled deployment within a body vessel.

In an embodiment, a method of controllably deploying an endovascular device comprises delivering, into a body vessel, a Nitinol structural element comprising a variable austenite finish temperature A_{f}(*x*) along a predetermined length (*L*) thereof, where 0 < *x* ≤ *L.* The variable austenite finish temperature A_{f}(*x*) increases or decreases monotonically as a function of x and lies above body temperature at any location along the predetermined length. After delivery into the body vessel, the Nitinol structural element is heated above body temperature. As the temperature of the Nitinol structural element reaches A_{f}(*x*) at each location *x* along the predetermined length, the Nitinol structural element recovers a pre-set shape at the respective location, and the endovascular device is controllably deployed.

In an embodiment, a method of heat setting an endovascular device for controlled deployment in a body vessel comprises securing a Nitinol structural element having a first end and a second end in a predetermined configuration and heating the first end of the Nitinol structural element. The second end of the Nitinol structural element does not undergo heating. During the heating of the first end, the temperature of the Nitinol structural element is increased along a length thereof by thermal conduction, producing a temperature gradient between the first end and the second end. After a predetermined time duration, the heating is halted, and the Nitinol structural element comprises a variable austenite finish temperature A_{f}(*x*) along the length (L) between the first end and the second end, where 0 < x ≤ *L.* The variable austenite finish temperature A_{f}(*x*) increases or decreases monotonically as a function of *x* and lies above body temperature at any location along the length. Thus, the endovascular device is configured for controlled deployment within a body vessel.

According to an aspect of the invention, there is provided a method of controllably deploying an endovascular device, the method comprising:
delivering a Nitinol structural element into a body vessel, the Nitinol structural element comprising a variable austenite finish temperature A_{f}(*x*) along a predetermined length (*L*) thereof, where 0 < *x* ≤ *L,* the variable austenite finish temperature A_{f}(*x*) increasing or decreasing monotonically as a function of x and being above body temperature at any location along the predetermined length; and
heating the Nitinol structural element above body temperature,
wherein, as a temperature of the Nitinol structural element reaches A_{f}(*x*) at each location along the predetermined length during the heating, the Nitinol structural element recovers a pre-set shape at the respective location and the endovascular device is controllably deployed.

In some embodiments, the Nitinol structural element further comprises a variable austenite start temperature Aₛ(*x*) having a value above body temperature at any location along the predetermined length.

In some embodiments, the variable austenite start temperature Aₛ(*x*) monotonically increases or decreases as a function of x.

In some embodiments, the heating is carried out uniformly along the predetermined length, the temperature of the Nitinol structural element being uniform to within ± 1°C.

In some embodiments, the heating is carried out by a heat source selected from the group consisting of: induction heater and resistive heater.

In some embodiments, a martensite start temperature of the Nitinol structural element is below body temperature, the deployed configuration remaining stable upon cooling after completion of the heating.

In some embodiments, the Nitinol structural element comprises a wire, rod, tube, or strip, and
the endovascular device comprises a stent, filter, cage, fastener, ratchet, or anchor.

In some embodiments, the Nitinol structural element comprises from about 50 at.% to about 52 at.% nickel.

### BRIEF DESCRIPTION OF THE DRAWINGS

Embodiments of the invention are described below, by way of example only, with reference to the accompanying drawings.
FIG. 1 illustrates controlled deployment of an exemplary endovascular device comprising a Nitinol structural element. The endovascular device is shown at the top of FIG. 1 in a delivery configuration and in the bottom-most schematic in a fully deployed configuration after heating. In between, the endovascular device is shown undergoing controlled deployment as a function of temperature.
FIG. 2 is a schematic of an induction triggered active anchoring system (ITAAS) configured to pierce a vessel wall and coil up, utilizing the controlled deployment process described herein.
FIG. 3 shows a heat setting simulation of a Nitinol wire of 0.48 mm in diameter heated at the first end (center of coil) to 500°C using a concentrated heat source. The coil is surrounded by air at 22°C.

### DETAILED DESCRIPTION

The present disclosure describes an endovascular device that can be delivered into a body vessel and can spontaneously recover - at a controlled rate - a deployed configuration when heated, preferably by a remote heat source. The method is enabled by the use of a Nitinol structural element having an austenite finish temperature (A_{f}) above body temperature that varies monotonically along a length of the element. The Nitinol structural element may take the form of a wire or another shape, such as a rod, tube or strip, preferably having an elongated geometry.

The Nitinol structural element comprises a nickel-titanium alloy that exhibits shape memory behavior. In other words, the nickel-titanium alloy can undergo a phase transformation that allows it to "remember" and return to a previous shape or configuration. More specifically, the nickel-titanium alloy can transform between a lower temperature phase (*e.g.,* martensite) and a higher temperature phase (*e.g.,* austenite) in order to effect shape or strain recovery. As would be known by the skilled artisan, austenite is characteristically the stronger phase, and martensite may be deformed up to a recoverable strain of about 8%. Strain introduced in the alloy in the martensitic phase may be substantially recovered upon completion of a reverse phase transformation to austenite, allowing the alloy to return to the previous shape. The temperature at which the strain recovery occurs may depend on the phase transformation temperatures of the nickel-titanium alloy, as discussed further below. The strain recovery can be driven by the application and removal of stress (superelastic effect) and/or by a change in temperature (shape memory effect), as in the present disclosure. Such alloys are commonly referred to as Nitinol or Nitinol alloys, and they are typically near-equiatomic in composition.

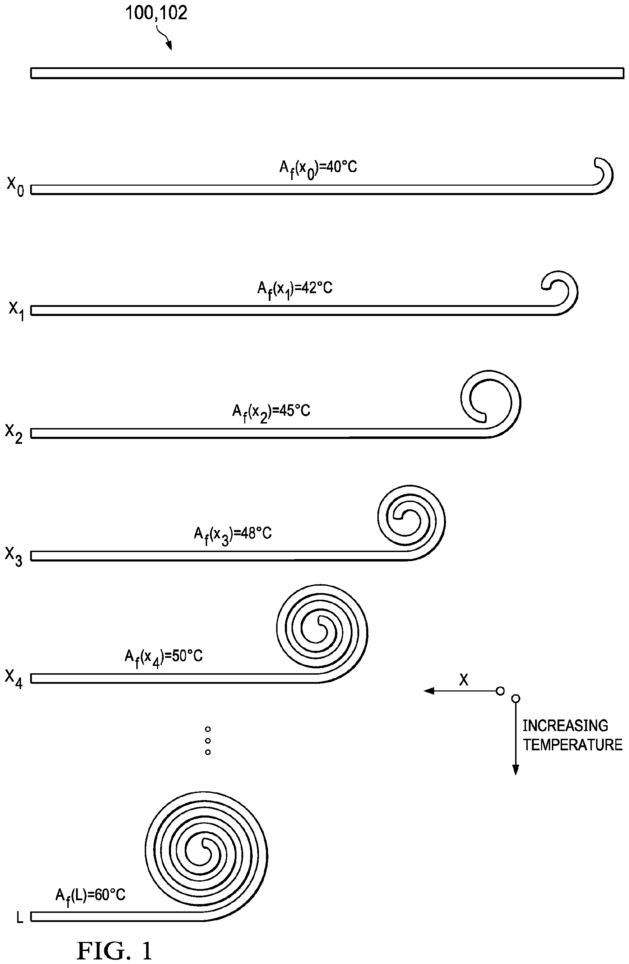

The method may be understood in view of the schematics of FIG. 1, which illustrate the controlled deployment of an exemplary endovascular device 100 comprising a Nitinol structural element (which is a Nitinol wire in this example) 102 during heating. The endovascular device 100 is shown at the top of FIG. 1 in a delivery configuration and in the bottom-most schematic in a fully deployed configuration after being heated to 60°C. In between, the endovascular device 100 is shown undergoing controlled deployment as the temperature of the Nitinol structural element 102 is increased above body temperature (37°C). The endovascular device 100 may comprise a stent, filter, cage, fastener, ratchet, anchor or another device.

The method entails delivering the Nitinol structural element 102 to a predetermined location in a body vessel. During delivery, the Nitinol structural element 102 is in an undeployed or delivery configuration which may be, for example, a substantially straight configuration that can be readily maneuvered through the vessel. As a consequence of the heat setting process described below, the Nitinol structural element 102 has a variable austenite finish temperature A_{f}(*x*) along a predetermined length (L) thereof, where 0 < x ≤ L. The variable austenite finish temperature A_{f}(*x*) is above body temperature (37°C) at any location along the predetermined length L and increases or decreases monotonically as a function of x. Thus, the endovascular device 100 is configured for gradual deployment after delivery and placement in the body vessel.

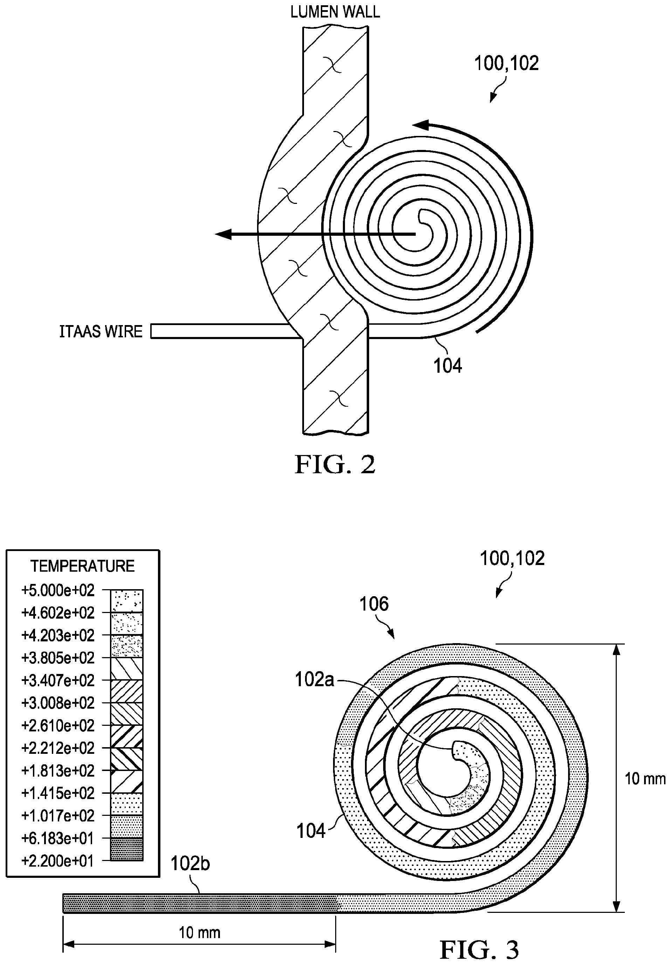

Table 1 summarizes the values of A_{f}(*x*) as a function of x for this example, where x has values *x₀, x₁, x₂, x₃, x₄* and *L*. Generally speaking, the variable austenite finish temperature may fall in a range from 37°C < A_{f}(*x*) ≤ Tₘₐₓ, where Tₘₐₓ is below a temperature that may be harmful to body tissue; for example, Tₘₐₓ may be 60°C or lower. The exemplary endovascular device 100 of this example is an anchoring device (e.g., an induction triggered active anchoring system (ITAAS)) configured to pierce the vessel wall and then coil up to exert an anchoring force, as shown schematically in FIG. 2 and described in U.S. Patent Application Serial No. 15/581,980, filed on April 28, 2017, which is hereby incorporated by reference.

**Table 1. Values of A_{f}(x) for Exemplary Endovascular Device as a Function of x**

| ***x*** | **A_{f}(*x*)** |
|---|---|
| *x*₀ | 40°C |
| *x*₁ | 42°C |
| *x*₂ | 45°C |
| *x₃* | 48°C |
| *x*₄ | 50°C |
| *L* | 60°C |

It is assumed that the Nitinol structural element 102, once placed in the body vessel, attains a temperature up to but not exceeding about 37°C, which is human body temperature. After placement in the vessel, the Nitinol structural element 102 is heated, preferably in a controlled manner (*e.g.,* at a specified heating rate), above body temperature in order to effect deployment. Typically, the heating begins only after the structural element 102 has reached a predetermined site in the body vessel, but in some cases heating may begin prior to reaching the predetermined site, e.g., during delivery. The heating may be carried out uniformly along the predetermined length L of the Nitinol structural element 102, such that the temperature is uniform to within ± 1°C.

As the temperature of the Nitinol structural element 102 is increased and reaches A_{f}(*x*) at each location (*e.g., x* = *x₀, x₁, x₂, x₃, x₄,* or *L*) along the predetermined length, the endovascular device 100 is gradually deployed. Gradual deployment occurs as the element 102 recovers a pre-set shape at each location during the heating. For example, once the temperature of the element rises from 37°C to 40°C, the tip region *x₀* of the Nitinol structural element 102 recovers a pre-set shape. As the heating continues and the temperature of the element reaches 42°C, the region of the element 102 between the tip region and up to position *x₁* recovers a pre-set shape. With further heating to a temperature of 45°C, the region of the element 102 between position *x₁* and up to position *x*₂ recovers a pre-set shape, and so on, as illustrated in FIG. 1. Ultimately, upon reaching a temperature at or above a highest value (60°C in this example) of the variable austenite finish temperature of the Nitinol structural element 102, the endovascular device 100 attains a fully deployed configuration. The heating of the Nitinol structural element 102 may be carried out by an external (*ex vivo*) or internal heat source, such as an induction heater or resistive heat source.

In addition to the variable austenite finish temperature A_{f}(*x*), the Nitinol structural element 102 may also have a variable austenite start temperature Aₛ(*x*) along the predetermined length that increases or decreases monotonically as a function of *x.* The variable austenite start temperature Aₛ(*x*) is preferably above body temperature at any location along the predetermined length to prevent deployment of the device from being initiated prematurely. As the temperature of the element reaches Aₛ(*x*) at each location *(e.g., x* = *x₀, x₁, x₂, x₃, x₄,* or *L*) along the predetermined length, shape recovery is initiated at that location.

As generally understood by those skilled in the art, austenite start temperature (Aₛ) refers to the temperature at which a phase transformation to austenite begins upon heating for a nickel-titanium shape memory alloy, and austenite finish temperature (A_{f}) refers to the temperature at which the phase transformation to austenite concludes. Martensite start temperature (Mₛ) refers to the temperature at which a phase transformation to martensite begins upon cooling for a nickel-titanium shape memory alloy, and martensite finish temperature (M_{f}) refers to the temperature at which the phase transformation to martensite concludes. Where the adjective "variable" appears in front of one of these terms, *e.g.,* "variable austenite start [finish] temperature," the term may be understood to refer to the temperature at which the phase transformation begins [concludes] for the nickel-titanium shape memory alloy as a function of *x* along the length of the element.

In order to maintain the deployed configuration of the endovascular device 100 after completion of the heating (*e.g.,* when the device has cooled to body temperature), it may be beneficial to ensure that the martensite start temperature of the Nitinol structural element 102 is below body temperature. With a martensite start temperature below body temperature, the shape memory alloy may remain austenitic (and thus in the deployed configuration) while deployed in the body, even after the heating is stopped. The martensite start temperature may also be selected to be below lower than body temperature, such as below room (ambient) temperature.

A method of heat setting a nitinol-based endovascular device for controlled deployment in a body vessel is set forth below in reference to FIG. 3, which shows a Nitinol structural element 102 having the form of a Nitinol wire 104 after a simulated heat treatment. The Nitinol structural element 102 has a first end 102a and a second end 102b and is secured in a predetermined configuration 106, which in the example of FIG. 3 is a coiled shape. The first end 102a of the element 102 that ultimately forms the center of the coiled shape (or "coil") may be fixed on a mandrel while ensuring that each loop of the coil is thermally isolated from adjacent loops. Thermal isolation may be achieved by incorporating an insulation layer and/or an air gap between the loops. In this example, the coil is surrounded by air at 22°C.

The first end 102a of the Nitinol structural element 102 is then heated or "heat set", while the second end 102b of the element 102 is not heated. In this example, the first end 102a is heated to 500°C by a concentrated heat source. During the heating, the temperature of the Nitinol structural element 102 is increased along a length (*L*) thereof by thermal conduction from the first end 102a, producing a (decreasing) temperature gradient between the first end 102a and the second end 102b, as illustrated in FIG. 3. As indicated above, an insulation layer and/or air gap may partially or fully cover the Nitinol structural element 102 along the length between the first end 102a and the second end 102b during the heating to provide thermal insulation between adjacent loops of the coil. The second end 102b of the element 102 may be actively cooled, e.g., by convective cooling, in order to modulate the temperature gradient along the length of the element 102. The heating may occur at a temperature ("heat setting temperature") and over a time duration sufficient to induce the Nitinol to adopt a "memory" of the predetermined configuration (coiled shape in this example) and a variable austenite finish temperature A_{f}(*x*) above body temperature along the length (*L*) between the first and second ends 102a, 102b, where 0 < x ≤ L. The variable austenite finish temperature A_{f}(*x*) monotonically increases or decreases as a function of *x* and is above body temperature at any location along the length L; thus, the endovascular device 100 is configured for gradual deployment within a body vessel.

It is recognized that the phase transformation temperatures of a nickel-titanium alloy, such as the austenite finish temperature, may be manipulated by altering the level of disclocations and/or the nickel content in solid solution, that is, the amount of nickel present in the matrix of the nickel-titanium alloy. The nickel content of the matrix may be controlled by either vaporization or traditional precipitation of nickel using a suitable heat treatment. Both the temperature and the duration of the heat treatment (e.g., heat setting), may influence the nickel content of the matrix.

Typically, heat setting temperatures from about 350°C to about 550°C are employed for the heating. Higher (or lower) temperatures within this temperature range and/or longer (or shorter) heat setting time durations may be used to increase or decrease the phase transformation temperatures. Guidance may be provided by a time-temperature-transformation (TTT) diagram for Nitinol, such as that set forth in Drexel et al., "The Effects of Cold Work and Heat Treatment on the Properties of Nitinol Wire," ASME 2007, 2nd Frontiers in Biomedical Devices Conference.

The heating of the first end 102a of the element 102 may be carried out using a concentrated heat source, such as a laser, resistive heating element or induction heater. After the predetermined time duration, the heating may be ceased and the Nitinol structural element 102 may optionally be exposed to a cooling fluid (*e.g.,* water) to rapidly quench the temperature. As a consequence of the heat setting, the Nitinol structural element 102 may have, in addition to a variable austenite finish temperature A_{f}(*x*), a variable austenite start temperature Aₛ(*x*), 0 < x ≤ *L,* that is also above body temperature. The variable austenite start temperature Aₛ(*x*) may increase or decrease monotonically as a function of *x.*

After heat setting, the Nitinol structural element 102 may be deformed (*e.g.,* straightened) into a delivery configuration for introduction into a body vessel. The deformation into the delivery configuration may occur while the shape memory alloy is in the martensitic phase. For example, the Nitinol structural element 102 may be cooled to a temperature at or below the martensite finish temperature, and the element 102 may be readily deformed to the desired delivery configuration. The Nitinol structural element 102 may remain in the delivery configuration until heated to a temperature at or above the lowest austenite start temperature (*e.g.,* Aₛ(*x₀*)) of the element, at which point deployment of the endovascular device 100 may be initiated. As explained above, the endoluminal medical device 100 deploys fully once heated at or above the highest austenite finish temperature (*e.g.,* A_{f}(*L*)), concluding the controlled deployment process.

Nitinol structural elements (*e.g.,* wire, rod, tubing, strip) 102 suitable for use in the present method may be obtained commercially from any of various vendors or fabricated from a nickel-titanium alloy ingot or billet of a suitable composition using mechanical working (*e.g.,* hot extrusion, cold drawing) and annealing methods known in the art. The nickel-titanium alloy is typically equiatomic or near-equiatomic in composition. For example, the nickel-titanium alloy may comprise from about 50 at.% Ni to about 52 at.% Ni, and titanium and any incidental impurities may account for the balance of the alloy. In some cases, the nickel-titanium alloy may also include a small amount of an additional alloying element (AAE) (e.g., from about 0.1 at.% AAE to about 10 at.% AAE) to enhance the superelastic or other properties of the nickel-titanium alloy. The additional alloying element may be selected from among B, Al, Cr, Mn, Fe, Co, Cu, Zn, Ga, Ge, Zr, Nb, Mo, Tc, Ru, Rh, Pd, Ag, Cd, In, Sn, Sb, Hf, Ta, W, Re, Os, Ir, Pt, Au, Hg, TI, Pb, Bi, Po, V, and Mischmetal.

Although embodiments of the present invention have been described in considerable detail, other embodiments are possible without departing from the present invention. The scope of the appended claims should not be limited, therefore, to the description of the preferred embodiments contained herein. All embodiments that come within the meaning of the claims, either literally or by equivalence, are intended to be embraced therein.

Furthermore, the advantages described above are not necessarily the only advantages of embodiments of the invention, and it is not necessarily expected that all of the described advantages will be achieved with every embodiment of the invention.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

The disclosures in United States patent application number 15/699,389, from which this application claims priority, and in the abstract accompanying this application are incorporated herein by reference.

## Claims

1. An endovascular device configured for controlled deployment in a body vessel, the endovascular device comprising:
a Nitinol structural element comprising a variable austenite finish temperature A_{f}(*x*) along a predetermined length (*L*) thereof, where 0 < x ≤ *L,* the variable austenite finish temperature A_{f}(*x*) monotonically increasing or decreasing as a function of x and being above body temperature at any location along the predetermined length, the endovascular device thereby being configured for controlled deployment within a body vessel.

2. The endovascular device of claim 1, wherein the endovascular device comprises a fully deployed configuration after being heated to a temperature at or above a highest value of the variable austenite finish temperature A_{f}(*x*).

3. The endovascular device of any preceding claim, wherein the Nitinol structural element further comprises a variable austenite start temperature Aₛ(*x*) above body temperature at any location along the predetermined length.

4. The endovascular device of claim 3, wherein the variable austenite start temperature Aₛ(*x*) monotonically increases or decreases as a function of x.

5. The endovascular device of any preceding claim, wherein the Nitinol structural element comprises from about 50 at.% to about 52 at.% nickel.

6. The endovascular device of any preceding claim, being a stent, filter, cage, fastener, ratchet or anchor.

7. A method of heat setting an endovascular device for controlled deployment in a body vessel, the method comprising:
securing a Nitinol structural element having a first end and a second end in a predetermined configuration;
heating the first end of the Nitinol structural element, the second end of the Nitinol structural element not being heated; and
after a predetermined time duration, halting the heating, wherein, during the heating, a temperature of the Nitinol structural element is increased along a length thereof by thermal conduction from the first end, thereby producing a temperature gradient between the first end and the second end,
wherein, after the heating, the Nitinol structural element comprises a variable austenite finish temperature A_{f}(*x*) along the length (*L*) between the first end and the second end, where 0 < *x* ≤ *L,* the variable austenite finish temperature A_{f}(*x*) increasing or decreasing monotonically as a function of *x* and being above body temperature at any location along the length, the endovascular device thereby being configured for controlled deployment within a body vessel.

8. The method of claim 7, wherein the Nitinol structural element is at least partially covered by an insulation layer between the first end and the second end during the heating.

9. The method of claim 7 or 8, wherein, after the heating, the Nitinol structural element comprises a variable austenite finish temperature Aₛ(*x*) along the length between the first end and the second end, where 0 < x ≤ *L,* the variable austenite start temperature Aₛ(*x*) increasing or decreasing monotonically as a function of *x* and being above body temperature at any location along the length.

10. The method of any of claims 7 to 9, further comprising, during the heating, cooling the second end of the Nitinol structural element to modulate the temperature gradient.

11. The method of any of claims 7 to 10, wherein the heating is carried out at a heat setting temperature from about 350°C to about 550°C using a concentrated heat source.

12. The method of any of claims 7 to 11, wherein halting the heating comprises quenching, the Nitinol structural element being exposed to a cooling fluid.
